# EUROPEAN PATENT SPECIFICATION

(11) **EP 3 565 524 B1**
(45) Date of publication and mention of the grant of the patent: **17.06.2026**
(21) Application number: 18701620.9
(22) Date of filing: 04.01.2018
(51) Int. Cl.: A61K 8/44, A61K 8/46, A61K 8/73, A61Q 19/00, A61K 8/81, A61Q 19/10, G01N 33/50, G01N 33/92

(54) **METHOD OF SELECTING MILD SKIN CLEANSERS**
VERFAHREN ZUR AUSWAHL VON MILDEN HAUTREINIGUNGSMITTELN
MÉTHODE DE SÉLECTION DE NETTOYANTS DOUX POUR LA PEAU

(30) Priority: 05.01.2017 US 201762442651 P
(43) Date of publication of application: 13.11.2019
(73) Proprietor: The Procter & Gamble Company, Cincinnati, OH 45202 (US)
(72) Inventor: WEI, Karl Shiqing, Cincinnati, Ohio 45202 (US)
(74) Representative: P&G Patent Germany
(86) International application number: PCT/US2018/012287
(87) International publication number: WO 2018/129119

(56) References cited:
- FR-A1- 2 792 728
- KR-A- 20170 102 857
- US-A1- 2002 182 112
- US-A1- 2011 257 030
- US-A1- 2012 009 285
- US-A1- 2013 253 057

## Description

### FIELD OF THE INVENTION

The present disclosure generally relates to methods of selecting skin cleansers and enhancing ceramide levels in skin.

### BACKGROUND OF THE INVENTION

Skin is a complex, multi-layered and dynamic system that provides a protective covering defining the interactive boundary between an organism and the environment. It is the largest organ of the body and is vitally important to both our health and our self-image. The skin comprises three principal layers, the epidermis, the dermis, and a layer of subcutaneous fat.

Adding to skin's complexity is the need to keep the skin clean. Skin cleansers have involved the utilization of soaps, body washes, and other personal cleansing compositions. Unfortunately, cleansers can dry out skin or exacerbate skin that is already dry. Because of the complexity of skin and the differences in the skin from season to season, it can be difficult to screen skin cleansing compositions to understand which ones will be milder.

US 2011/ 0257030 A1 refers to methods for selecting and formulating compositions for treating and maintaining the quality of skin in a select population, wherein a composition is selected for use in a personal care product based on its demonstrated biological effect to improve skin quality in the population as evidenced by one or more biomarker changes that correlate with improvement as evidenced by one or more obj ective measurements of skin health in the population.

As such, there is a need for improved methods to screen skin cleansing compositions.

### SUMMARY OF THE INVENTION

A method of screening cleansers for mildness, wherein mildness results in enhancing selective ceramides in the stratum corneum for enhanced skin barrier function and hydration is provided and comprises:
a) measuring the level of one or more ceramides on an area of skin prior to application of a cleanser, wherein the ceramide comprises N₂₄P₁₈, N₂₆DS₁₈, or N₂₈P₁₈;;
b) applying the cleanser to the area of skin for 21 days, wherein 0.07mL or more of the cleanser is applied during each application, wherein the cleanser comprises sodium trideceth-2 sulfate, cocamidopropyl betaine, guar hydroxypropyltrimonium chloride, a C10-C30 acrylate crosspolymer, and a benefit agent;
c) measuring the level of one or more ceramides after the product application of 21 days on the area of skin, according to the Analysis of Skin Lipids from D-Squame Discs as disclosed herein;
wherein the cleanser is mild if the level of the one or more ceramides is at least 10%vs. the no treatment control.

### BRIEF DESCRIPTION OF THE DRAWINGS

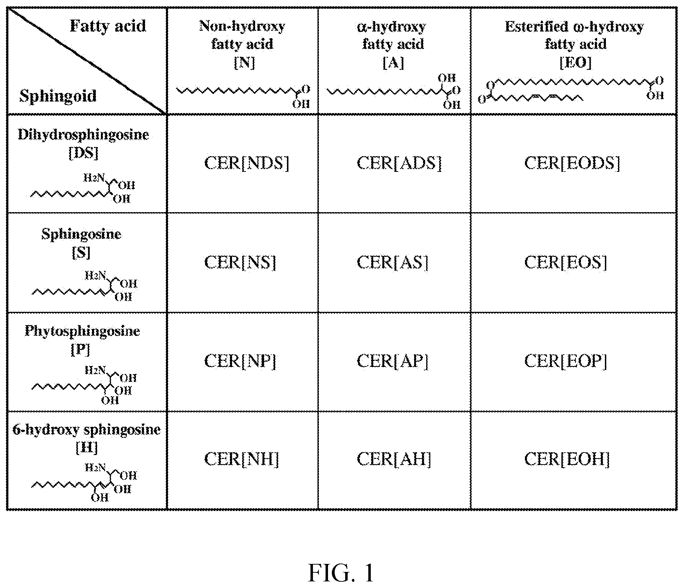

FIG. 1 is table showing the varying combinations of fatty acid and sphingoid in ceramides.

### DETAILED DESCRIPTION OF THE INVENTION

It is believed the scope of the present invention will be better understood from the following description.

The devices, apparatuses, methods, components, and/or compositions of the present invention can include, consist essentially of, or consist of, the components of the present invention as well as other ingredients described herein. As used herein, "consisting essentially of" means that the devices, apparatuses, methods, components, and/or compositions may include additional ingredients, but only if the additional ingredients do not materially alter the basic and novel characteristics of the claimed devices, apparatuses, methods, components, and/or compositions.

All percentages and ratios used herein are by weight of the total composition and all measurements made are at 25°C, unless otherwise designated.

All measurements used herein are in metric units unless otherwise specified.

### I. Definitions

As used herein, the following terms shall have the meaning specified thereafter:
"Associative polymer" refers to a water-dispersible polymer comprising hydrophobic groups at an end or pendants to a hydrophilic backbone.
"Non-associative polymer" refers to a water-dispersible polymer with a relatively uniform hydrophilic backbone lacking hydrophobic groups.
"STnS" refers to sodium trideceth(n) sulfate, wherein n can define the average number of moles of ethoxylate per molecule.

The phrase "substantially free of" as used herein, unless otherwise specified, means that the personal care composition comprises less than 2%, less than 1%, less than about0.5%, or even less than 0.1% of the stated ingredient. The term "free of", as used herein, means that the personal care composition comprises 0% of the stated ingredient that is the ingredient has not been added to the personal care composition. However, these ingredients may incidentally form as a byproduct or a reaction product of the other components of the personal care composition.

"Visually distinct" generally refers to a region of the multiphase personal care composition having one average composition, as distinct from another region having a different average composition, wherein the regions can be visible to the unaided naked eye. This would not preclude distinct regions from comprising two similar multiphase personal care compositions or phases where one multiphase personal care composition or phase can comprise certain pigments, dyes, particles, and various optional ingredients, hence providing a region of different average composition (e.g., different textures or different colors).

Ceramides are a family of lipid molecules that makeup part of the stratum corneum layer of the skin. Together with cholesterol and saturated fatty acids, ceramides help the skin to be water-impermeable to help prevent water loss and also as a protective layer to keep unwanted microorganisms from entering the body through the skin. When the ceramide level of skin is suboptimal, the stratum corneum can become compromised. The skin can also become dry and irritated.

Ceramides are composed of a fatty acid chain amide linked to a sphingoid base. There are three types of fatty acids which can be part of a ceramide. These are non-hydroxy fatty acids (N), α-hydroxy fatty acids (A), and esterified ω-hydroxy fatty acids (EO). In addition, there are four sphingoid bases: dihydrosphingosine (DS), sphingosine (S), phytosphingosine (P), and 6-hydroxy sphingosine (H). This makes for a total of 12 classes of ceramides, see FIG. 1. Within each class of ceramides, there are ceramides of various chain lengths, depending on the number of sphingoid side chains (or the length of the fatty acid, or both). For example, in the ω-hydroxy fatty acid sphingoid class (EOS), there are C₃₀C₁₈₍₁₎, C₃₀C₁₈₍₂₎, C₃₂C₁₈₍₁₎, C₃₂C₁₈₍₂₎, etc. In addition, the varying classes can be divided into long chain and short chain. The ω-hydroxy fatty acid sphingoid class (EOS), non-hydroxy fatty acid dihydrosphingosine class (NDS), and the non-hydroxy fatty acid phytosphingosine class (NP) are considered long chain, while the α-hydroxy fatty acid sphingosine class (AS), α-hydroxy fatty acid 6-hydroxy sphingosine (AH), and α-hydroxy fatty acid phytosphingosine (AP) are considered short chain.

While reviewing information on seasonal impact on skin, a trend was discovered. Certain ceramide levels fluctuate between the summer and winter months. This is especially true for the longer chain ceramides like, EOS, NDS, and NP. As can be seen in Table 1 below, ceramides from the EOS class that were evaluated ranged from a summer to winter index (summer value / winter value) of 1.82 to 2.19. This means that there was an increase of 80% or higher of the Ceramide EOS class in the summer season as compared to the winter season. One hypothesis is that these specific ceramide types play more important role in the enhanced barrier function and skin hydration during the summer season as compared to the winter season.

**Table 1**

| Ceramide | Class | Summer | Winter | Summer/Winter |
|---|---|---|---|---|
| C30_C18_1 | Ceramide-EOS | 0.31 | 0.17 | 1.82 |
| C30_C18_2 | Ceramide-EOS | 2.08 | 0.95 | 2.19 |
| C32_C18_1 | Ceramides-EOS | 0.23 | 0.11 | 2.09 |
| C32_C18_2 | Ceramide-EOS | 1.11 | 0.56 | 1.98 |
| N24_0_DS18 | Ceramide-NDS | 1.46 | 0.87 | 1.68 |
| N24_0_P18 | Ceramide-NP | 6.59 | 3.56 | 1.85 |
| N26_0_DS18 | Ceramide-NDS | 2.90 | 1.85 | 1.57 |
| N26_0_P18 | Ceramide-NP | 6.38 | 3.31 | 1.93 |
| N28_0_P18 | Ceramide-NP | 9.11 | 4.57 | 1.99 |
| N30_0_P18 | Ceramide-NP | 5.06 | 2.34 | 2.16 |

Short chain ceramides are also impacted by seasonal effect, but for the most part, not to the extent of the longer chain. As can be seen in Table 2 below, the impact to all but the AP class is less than that of the longer chain ceramides.

**Table 2**

| Ceramide | Class | Summer | Winter | Summer/Winter |
|---|---|---|---|---|
| A16_0_S18 | Ceramide-AS | 1.50 | 1.31 | 1.15 |
| A24_0_H18 | Ceramide-AH | 1.23 | 0.96 | 1.28 |
| A24_0_P18 | Ceramide-AP | 0.93 | 0.52 | 1.79 |
| A26_0_H18 | Ceramide-AH | 2.30 | 1.67 | 1.38 |
| A26_0_P18 | Ceramide-AP | 1.28 | 0.72 | 1.78 |

Thus, it is believed that the reduction in at least some of the ceramides (especially the long chain ceramides) may contribute to seasonal dry skin.

Skin cleansers can also contribute to skin dryness as the surfactants or soaps utilized within these types of products necessarily remove some of the sebum naturally occurring on the skin. Some cleansers can be formulated to replace the removed sebum with a moisturizer, like petrolatum. These types of products can not only mitigate the drying impact of the surfactant or soap, but in some cases can even positively impact the moisture of the skin such that it is better than before use.

Now, with the understanding above with respect to ceramides, a skin cleanser can be formulated to not only minimize its negative impact on some of the ceramides, but to enhance the selective ceramides in the stratum corneum for enhanced skin barrier function and hydration through the design of a skin cleanser. This also allows for such formulations to be screened for skin mildness and barrier improvement. This could be done, for example, by having subjects use the body wash and measuring the impact on ceramide levels, particularly the longer chain ceramides, like EOS. For example, one could utilize the Dry Skin Grade Screen and Application of Materials Method below for 7 days to 21 days, and measure the ceramide level before the initial application on day 1 and on the 21st day. Ceramide levels can be measured by standard analytical methods. One method is to analyze the selected ceramides from extracts of D-Squame^{®} discs sampled from human skin using gradient supercritical fluid chromatography (SFC) with tandem mass spectrometry (MS/MS) with detection in the positive and negative ionization modes depending on the analyte using atmospheric pressure chemical ionization (APCI). It is preferred to combine two tapes from each subject for enhanced sensitivity. Two tape strips from each subject were transferred to 20 mL glass vials, spiked with an internal standard mixture (D₆-cholesterol, D₇-cholesterol sulfate, D₄₇-tetradecanoic acid, D₃-heptadecanoic acid, D₇-sphinanine and D₃₁-N-palmitoyl-1-D-eryhhro-sphingosine (D₃₁ Ceramide)) and extracted using 3 mL of methanol with sonication at ambient temperature. The vials were centrifuged and the methanol layer removed and placed in separate glass vial. The tape strips were then extracted with 3 mL of hexane with sonication for 15 min at ambient temperature and the hexane layer was isolated. The hexane and methanol layers for each set of tapes were then combined, dried under nitrogen at 50°C and finally reconstituted in chloroform:MeOH (3:1; v/v). Standards (myristic acid, palmitic acid, palmitoleic acid, octadecanoic acid, oleic acid, linoleic acid, docosanoic acid, tetraocosanoic acid, cholesterol, cholesterol sulfate, N(24_0)P(18), N(24_0)DS(18), A(16_0)S(18), A(24_0) P(18), ceramide EOS-C30, S(18)) were prepared in chloroform:MeOH (3:1) over a range of appropriate concentrations. The standards, spiked with internal standard, and the reconstituted samples were analyzed by gradient SFC with MS/MS detection using APCI. The selected ceramides were monitored in the positive ion mode. The peak area ratio (standard peak area/internal standard peak area) for each standard level were used to construct a linear regression curve for each of the standard analytes. The lipid mass found for each analyte was divided by the total protein (based on the standard BCA method) as the normalized ceramide content. The selected ceramides that can be used to evaluate the performance a skin cleanser include Ceramide NP N₂₄P₁₈, Ceramide NDS N₂₆DS₁₈, Ceramide NP N₂₈P₁₈.

### SKIN CLEANSING COMPOSITIONS

A skin cleansing composition can include a cleansing phase and a benefit phase, where the cleansing phase can be structured. The cleansing phase and the benefit phase can be in physical contact. The phases may be blended or mixed to a significant degree, but still be physically distinct such that the physical distinctiveness is undetectable to the naked eye. The phases can also be made to occupy separate and distinct physical spaces inside a package in which the phases can be stored. **In** such an arrangement, the cleansing phase and the benefit phase can be stored such that the phases are not in direct contact with one another. The cleaning phase and the benefit phase can be in physical contact while remaining visibly distinct to give, for example, a striped or marbled configuration. The phases may be stable, meaning, if they are distinct phases they stay distinct over the shelf life of the product and if they are blended, they stay blended with no major separation of the phases upon sitting during the shelf life of the product.

The skin cleansing composition can include a combination of one or more of the above multiphase skin cleansing compositions. For example, one blended multiphase skin cleansing composition can be stacked as stripes with another blended multiphase skin cleansing composition.

### Cleansing Phase

The skin cleansing composition can include a cleansing phase. The cleansing phase can comprise as least one anionic surfactant. The cleansing phase may contain from 3% to 20%, from 5% to 15%, from 7% to 15%, from 5% to 13%, from 5% to 20%, or any combination of the upper, lower, and included limits within the ranges 2% to 30%, of surfactant, by weight of the skin cleansing composition.

The cleansing phase may comprise a structured domain. The structured domain can be, for example, an opaque structured domain, which can be a lamellar phase. A lamellar phase can provide resistance to shear, adequate yield to suspend particles and droplets while providing long term stability because it is thermodynamically stable. The lamellar phase tends to have a viscosity that minimizes the need for viscosity modifiers, but they can be included if desired. The cleaning phase may comprise more than one surfactant.

The anionic surfactants can be either linear or branched. Examples of some suitable linear anionic surfactants include ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium laureth sulfate, potassium laureth sulfate, sodium lauryl sarcosinate, sodium lauryl sulfate, sodium lauroyl sarcosinate, lauryl sarcosine, cocoyl sarcosine, ammonium cocoyl sulfate, sodium cocoyl isethionate, ammonium lauroyl sulfate, sodium cocoyl sulfate, sodium lauroyl sulfate, potassium cocoyl sulfate, potassium lauryl sulfate, monoethanolamine cocoyl sulfate, sodium tridecyl benzene sulfonate, sodium dodecyl benzene sulfonate, and combinations thereof.

Examples of some suitable branched anionic surfactants include but are not limited to the following surfactants: sodium trideceth sulfate, sodium tridecyl sulfate, sodium C₁₂₋₁₃ alkyl sulfate, sodium C₁₂₋₁₅ alkyl sulfate, sodium C₁₁₋₁₅ alkyl sulfate, sodium C₁₂₋₁₈ alkyl sulfate, sodium C₁₀₋₁₆ alkyl sulfate, sodium C₁₂₋₁₃ pareth sulfate, sodium C₁₂₋₁₃ pareth-n sulfate, sodium C₁₂₋₁₄ pareth-n sulfate, and combinations thereof. Other salts of all the aforementioned surfactants are useful, such as TEA, DEA, ammonia, and potassium salts. Useful alkoxylates include the ethylene oxide (EO), propylene oxide (PO) and EO/PO mixed alkoxylates. Phosphates, carboxylates and sulfonates prepared from branched alcohols are also useful anionic branched surfactants. Branched surfactants can be derived from synthetic alcohols such as the primary alcohols from the liquid hydrocarbons produced by Fischer-Tropsch condensed syngas, for example Safol^{™} 23 Alcohol available from Sasol North America, Houston, TX; from synthetic alcohols such as Neodol^{™} 23 Alcohol available from Shell Chemicals, USA; from synthetically made alcohols such as those described in U.S. Patent No. 6,335,312 issued to Coffindaffer, et al on January 1, 2002. Suitable examples of alcohols are Safol^{™} 23 and Neodol^{™} 23. Suitable examples of alkoxylated alcohols are Safol^{™} 23-3 and Neodol^{™} 23-3. Sulfates can be prepared by conventional processes to high purity from a sulfur based SO₃ air stream process, chlorosulfonic acid process, sulfuric acid process, or Oleum process. Preparation via SO₃ air stream in a falling film reactor is a preferred sulfation process.

The anionic surfactant may also be STnS, wherein n can define the average moles of ethoxylation. A cleansing phase can include from 5% to 20%, from 7% to 18%, from 9% to 16%, from 11% to 14%, by weight of the skin cleansing composition, of STnS. A structured cleansing phase can include from 5% to 20%, from 7% to 18%, from 9% to 16%, from 11% to 14%, by weight of the skin cleansing composition, of STnS. n can range from 0 to 3, from 0.5 to 2.7, from 1.1 to 2.5, from 1.8 to 2.2, or n can be 2. When n is less than 3, STnS can provide improved stability, improved compatibility of benefit agents within the skin cleansing compositions, and increased mildness of the skin cleansing composition. Such described benefits of STnS are disclosed in U.S. Patent Application Publication No. 2012/0009285.

Further, the cleansing phase can comprise a structuring system wherein the structuring system can comprise an associative polymer and a non-associative polymer. The structuring system can comprise from 0.01% to 5%, from 0.05% to 1%, from 0.07% to 0.5%, or from 0.1% to 0.3%, by weight of the skin cleansing composition, of a non-associative polymer. The structuring system can also comprise from 0.01% to 5%, from 0.05% to 1%, from 0.07% to 0.5%, or from 0.1% to 0.3%, by weight of the skin cleansing composition, of a non-associative polymer. The structuring system can comprise from 0.001% to 5%, from 0.005% to 0.5%, from 0.007% to 0.05%, from 0.008% to 0.04%, or from 0.01% to 0.03%, by weight of the skin cleansing composition, of an associative polymer. The structuring system can comprise from 0.001% to 5%, from 0.005% to 0.5%, from 0.007% to 0.05%, from 0.008% to 0.04%, or from 0.01% to 0.03%, by weight of the skin cleansing composition, of an associative polymer. As noted herein, stability of a skin cleansing composition can be maintained or enhanced even with the reduction of associative polymer with the addition of a non-associative polymer.

Such associative polymers can be crosslinked, alkali swellable, associative polymers comprising acidic monomers and associative monomers with hydrophobic end groups, whereby the associative polymer comprises a percentage hydrophobic modification and a hydrophobic side chain comprising alkyl functional groups. Without intending to be limited by theory, it is believed the acidic monomers can contribute to an ability of the associative polymer to swell in water upon neutralization of acidic groups; and associative monomers anchor the associative polymer into structured surfactant hydrophobic domains, e.g., lamellae, to confer structure to the surfactant phase and keep the associative polymer from collapsing and losing effectiveness in the presence of an electrolyte. The crosslinked, associative polymer can comprise a percentage hydrophobic modification, which is a mole percentage of monomers expressed as a percentage of a total number of all monomers in a polymer backbone, including both acidic and other non-acidic monomers. Percentage hydrophobic modification of the associative polymer, hereafter %HM, can be determined by the ratio of monomers added during synthesis or by analytical techniques such as proton nuclear magnetic resonance (NMR). Associative alkyl side chains can comprise, for example, butyl, propyl, stearyl, steareth, cetyl, lauryl, laureth, octyl, behenyl, beheneth, steareth, or other linear, branched, saturated, or unsaturated alkyl or alketh hydrocarbon side chains.

Crosslinked, associative polymers having certain %HM and certain carbon numbers of hydrophobic end groups of alkyl side chains can provide significant enhancement of structure to skin cleansing compositions comprising a structured surfactant, especially to skin cleansing compositions comprising reduced levels of surfactant. Such associative polymers can also provide the above structure at surprisingly low levels of polymer structurant. Concentrations of associative polymers of up to 5% or even 10% have been known to provide a sufficient amount structure (e.g., exemplary compositions of U.S. Patent Nos. 7,119,059 (Librizzi, et al.) and 6,897,253 (Schmucker-Castner, et al.). It has been discovered that when an associative polymer %HM and an alkyl side chain number of carbons can be optimized, the structure of an aqueous structured surfactant phase can be increased using only less than 3 wt%, less than 2%, less than 1%, and less than 0.2%, of an associative polymer, as a percentage of an aqueous structured surfactant phase.

The acidic monomer can comprise any acid functional group, for example sulfate, sulfonate, carboxylate, phosphonate, or phosphate or mixtures of acid groups. The acidic monomer can comprise, for example, a carboxylate. Alternatively, the acidic monomer can be an acrylate, including acrylic acid and/or methacrylic acid. The acidic monomer can comprise a polymerizable structure, e.g., vinyl functionality. Mixtures of acidic monomers, for example acrylic acid and methacrylic acid monomer mixtures, may be useful as well.

The associative monomer can comprise a hydrophobic end group and a polymerizable component, e.g., vinyl, which can be attached. The hydrophobic end group can be attached to the polymerizable component, hence to the polymer chain, by different means but can be attached by an ether or ester or amide functionality, such as an alkyl acrylate or a vinyl alkanoate monomer. The hydrophobic end group can also be separated from the chain, for example, by an alkoxy ligand such as an alkyl ether. The associative monomer can be, for example, an alkyl ester, an alkyl (meth)acrylate, where (meth)acrylate is understood to mean either methyl acrylate or acrylate, or mixtures of the two.

Sometimes, the hydrophobic end group of the associative polymer can be incompatible with the aqueous phase of the skin cleansing composition and can associate with lathering surfactant hydrophobe components. Without intending to be limited by theory, it is believed that longer alkyl chains of structuring polymer hydrophobe end groups can increase incompatibility with the aqueous phase to enhance structure, whereas shorter alkyl chains having carbon numbers closely resembling lathering surfactant hydrophobes (e.g., 12 to 14 carbons) or multiples thereof (for bilayers, e.g.) can also be effective. An ideal range of hydrophobic end group carbon numbers combined with an optimal percentage of hydrophobic monomers expressed as a percentage of the polymer backbone can provide increased structure to the skin cleansing composition comprising a structured surfactant with low levels of polymer structurant.

An exemplary associative polymer can include AQUPEC^{®} SER-300 made by Sumitomo Seika of Japan, which is an acrylate/C₁₀-C₃₀ alkyl acrylate cross-polymer and comprises stearyl side chains with less than 1% HM. Associative polymers can comprise C₁₆ (cetyl) alkyl hydrophobic side chains with 0.7% hydrophobic modification, but a percentage hydrophobic modification can be up to an aqueous solubility limit in surfactant compositions (e.g., up to 2%, 5%, or 10%). Other associative polymers can include stearyl, octyl, decyl and lauryl side chains, alkyl acrylate polymers, polyacrylates, hydrophobically-modified polysaccharides, hydrophobically-modified urethanes, AQUPEC^{®} SER-150 (acrylate/C₁₀-C₃₀ alkyl acrylate cross-polymer) comprising C₁₈ (stearyl) side chains and 0.4% HM, and AQUPEC^{®} HV-701EDR which comprises C₈ (octyl) side chains and 3.5% HM, and mixtures thereof. Another exemplary associative polymer can be Stabylen 30 manufactured by 3V Sigma S.p.A., which has branched isodecanoate hydrophobic associative side chains.

As set forth above, the cleansing phase of a skin cleansing composition can further include a non-associative polymer. Suitable non-associative polymers can include water-dispersible polymers with relatively uniform hydrophilic backbone lacking hydrophobic groups. Examples of non-associative polymers can include biopolymer polysaccharides (e.g., xanthan gum, gellan gum), cellulosic polysaccharides (e.g., carboxymethyl cellulose, carboxymethyl hydroxyethyl cellulose), other polysaccharides (e.g., guar gum, hydroxypropyl guar, and sodium alginate), and synthetic hydrocarbon polymers (e.g., polyacrylamide and copolymers, polyethylene oxide, polyacrylic acid copolymers).

Skin cleansing compositions can additionally comprise an organic cationic deposition polymer in one or more phases as a deposition aid for the benefit agents described herein. Suitable cationic deposition polymers can contain cationic nitrogen-containing moieties such as quaternary moieties. Non-limiting examples of cationic deposition polymers can include polysaccharide polymers, such as cationic cellulose derivatives. Cationic cellulose polymers can be salts of hydroxyethyl cellulose reacted with trimethyl ammonium substituted epoxide, referred to in the industry (CTFA) as Polyquaternium 10, which can be available from Amerchol Corp. (Edison, N.J.) in their Polymer KG, JR, and LR series of polymers. Other suitable cationic deposition polymers can include cationic guar gum derivatives, such as guar hydroxypropyltrimonium chloride, specific examples of which can include the Jaguar series commercially available from Rhodia Inc. and N-Hance polymer series commercially available from Aqualon. Deposition polymers can have a cationic charge density from 0.8 meq/g to 2.0 meq/g or from 1.0 meq/g to 1.5 meq/g.

The skin cleansing composition can be optionally free of or substantially free of sodium lauryl sulfate, hereinafter SLS, and/or ammonium lauryl sulfate, hereinafter ALS, and can comprise at least a 70% lamellar structure. However, in an alternative arrangement, the cleansing phase can comprise at least one surfactant, wherein the at least one surfactant includes SLS and/or ALS. Suitable examples of SLS are described in U.S. Patent Application Serial No. 12/817,786.

A skin cleansing composition can further comprise from 0.1% to 20%, by weight of the skin cleansing composition, of a cosurfactant. The cosurfactant can comprise amphoteric surfactants, zwitterionic surfactants, or mixtures thereof. For example, a skin cleansing composition can include an amphoteric surfactant and/or a zwitterionic surfactant. Suitable amphoteric or zwitterionic surfactants can include those described in U.S. Patent Nos. 5,104,646 and 5,106,609.

Amphoteric surfactants can include those that can be broadly described as derivatives of aliphatic secondary and tertiary amines in which an aliphatic radical can be a straight or branched chain and wherein an aliphatic substituent can contain from 8 to 18 carbon atoms such that one carbon atom can contain an anionic water solubilizing group, e.g., carboxy, sulfonate, sulfate, phosphate, or phosphonate. Examples of compounds falling within this definition can be sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Pat. No. 2,658,072, N-higher alkyl aspartic acids such as those produced according to the teaching of U.S. Pat. No. 2,438,091, and products described in U.S. Pat. No. 2,528,378. Other examples of amphoteric surfactants can include sodium lauroamphoacetate, sodium cocoamphoactetate, disodium lauroamphoacetate disodium cocodiamphoacetate, and mixtures thereof. Amphoacetates and diamphoacetates can also be used.

Zwitterionic surfactants suitable for use can include those that are broadly described as derivatives of aliphatic quaternary ammonium, phosphonium, and sulfonium compounds, in which aliphatic radicals can be straight or branched chains, and wherein an aliphatic substituent can contain from 8 to 18 carbon atoms such that one carbon atom can contain an anionic group, e.g., carboxy, sulfonate, sulfate, phosphate, or phosphonate. Other zwitterionic surfactants can include betaines, including cocoamidopropyl betaine.

Other suitable surfactants or cosurfactants that can generally be used in a structured cleansing phase for a skin cleansing composition are described in McCutcheon's: Detergents and Emulsifiers North American Edition (Allured Publishing Corporation 1947) (1986), McCutcheon's, Functional Materials North American Edition (Allured Publishing Corporation 1973) (1992) and U.S. Patent No. 3,929,678 (filed Aug. 1, 1974).

The cleansing phase of the skin cleansing composition can also comprise water. The structured cleansing phase of the skin cleansing composition can comprise from 10% to 90%, from 40% to 85%, or from 60% to 80%, by weight of the skin cleansing composition, of water.

Other optional additives can be included in the cleaning phase, including, for example, an emulsifier (e.g., non-ionic emulsifier) and electrolytes. Suitable electrolytes can include anions such as phosphate, chloride, sulfate, citrate, and mixtures thereof and cations such as sodium, ammonium, potassium, magnesium, and mixtures thereof. For example, suitable electrolytes can include sodium chloride, ammonium chloride, sodium sulfate, ammonium sulfate, and mixtures thereof. Other suitable emulsifiers and electrolytes are described in U.S. Patent Publication No. 2012/0009285.

### Benefit Phase

As noted herein, skin cleansing compositions can include a benefit phase. The benefit phase can be hydrophobic and/or anhydrous. The benefit phase can also be substantially free of or free of surfactant.

The benefit phase can also include one or more benefit agents. In particular, the benefit phase can comprise from 0.1% to 50%, by weight of the skin cleansing composition, of a benefit agent. In other arrangements, the benefit phase can include from 0.5% to 20%, by weight of the skin cleansing composition, of the benefit agent. Examples of such benefit agents can include petrolatum, glyceryl monooleate, mineral oil, natural oils (e.g., soybean oil), and mixtures thereof.

Benefit agents can include water insoluble or hydrophobic benefit agents. Additional examples of benefit agents can include SEFOSE^{®}, lanolin, lanolin derivatives, lanolin esters, lanolin oil, natural waxes, synthetic waxes, volatile organosiloxanes, derivatives of volatile organosiloxanes, non-volatile organosiloxanes, derivatives of non-volatile organosiloxanes, natural triglycerides, synthetic triglycerides, and combinations thereof. Other suitable benefit agents are described in U.S. Patent Application Serial No. 13/157,665.

SEFOSE^{®} includes one or more types of sucrose polyesters. Sucrose polyesters are derived from a natural resource and therefore, the use of sucrose polyesters as the benefit agent can result in a positive environmental impact. Sucrose polyesters are polyester materials having multiple substitution positions around the sucrose backbone coupled with the chain length, saturation, and derivation variables of the fatty chains. Such sucrose polyesters can have an esterification ("IBAR") of greater than 5. For example, the sucrose polyester may have an IBAR of 5 to 8. In another example, the sucrose polyester may have an IBAR of 5-7; in another example, the sucrose polyester can have an IBAR of 6. In yet another example, the sucrose polyester can have an IBAR of 8. As sucrose polyesters can be derived from natural resources, a distribution in the IBAR and chain length may exist. For example, a sucrose polyester having an IBAR of 6 may contain a mixture of mostly IBAR of 6, with some IBAR of 5, and some IBAR of 7. Additionally, such sucrose polyesters may have a saturation or iodine value ("IV") of 3 to 140. In another example, the sucrose polyester may have an IV of 10 to 120. In yet another example, the sucrose polyester may have an IV of 20 to 100. Further, such sucrose polyesters may have a chain length of C₁₂ to C₂₀.

Non-limiting examples of sucrose polyesters suitable for use include SEFOSE^{®} 1618S, SEFOSE^{®} 1618U, SEFOSE^{®} 1618H, Sefa Soyate IMF 40, Sefa Soyate LP426, SEFOSE^{®} 2275, SEFOSE^{®} C1695, SEFOSE^{®} C18:0 95, SEFOSE^{®} C1495, SEFOSE^{®} 1618H B6, SEFOSE^{®} 1618S B6, SEFOSE^{®} 1618U B6, Sefa Cottonate, SEFOSE^{®} C1295, Sefa C895, Sefa C1095, SEFOSE^{®} 1618S B4.5, all available from The Procter and Gamble Co. of Cincinnati, Ohio. Sucrose polyesters can also be combined with other benefit agents in the benefit phase.

Non-limiting examples of glycerides suitable for use as hydrophobic benefit agents herein can include castor oil, safflower oil, corn oil, walnut oil, peanut oil, olive oil, cod liver oil, almond oil, avocado oil, palm oil, sesame oil, soybean oil, vegetable oils, sunflower seed oil, vegetable oil derivatives, coconut oil and derivatized coconut oil, cottonseed oil and derivatized cottonseed oil, jojoba oil, cocoa butter, petrolatum, mineral oil, and combinations thereof.

Non-limiting examples of alkyl esters suitable for use as hydrophobic benefit agents herein can include isopropyl esters of fatty acids and long chain esters of long chain (i.e. C₁₀-C₂₄) fatty acids, e.g., cetyl ricinoleate, non-limiting examples of which can include isopropyl palmitate, isopropyl myristate, cetyl ricinoleate, and stearyl ricinoleate. Other examples can include hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof.

Non-limiting examples of alkenyl esters suitable for use as hydrophobic benefit agents herein can include oleyl myristate, oleyl stearate, oleyl oleate, and combinations thereof.

Non-limiting examples of polyglycerin fatty acid esters suitable for use as hydrophobic benefit agents herein can include decaglyceryl distearate, decaglyceryl diisostearate, decaglyceryl monomyriate, decaglyceryl monolaurate, hexaglyceryl monooleate, and combinations thereof.

Non-limiting examples of lanolin and lanolin derivatives suitable for use as hydrophobic benefit agents herein can include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol ricinoleate, and combinations thereof.

Non-limiting examples of silicone oils suitable for use as hydrophobic benefit agents herein can include dimethicone copolyol, dimethylpolysiloxane, diethylpolysiloxane, mixed C₁-C₃₀ alkyl polysiloxanes, phenyl dimethicone, dimethiconol, and combinations thereof. Non-limiting examples of silicone oils useful herein are described in U.S. Patent No. 5,011,681. Still other suitable hydrophobic skin benefit agents can include milk triglycerides (e.g., hydroxylated milk glyceride) and polyol fatty acid polyesters.

Additional optional materials can also be added to the skin cleansing composition to treat the skin, or to modify the aesthetics of the skin cleansing composition as is the case with perfumes, colorants, dyes, or the like. Optional materials useful in products herein can be categorized or described by their cosmetic and/or therapeutic benefit or their postulated mode of action or function. However, it can be understood that actives and other materials useful herein can, in some instances, provide more than one cosmetic and/or therapeutic benefit or function or operate via more than one mode of action. Therefore, classifications herein can be made for convenience and cannot be intended to limit a material to a particularly stated application or applications listed. A precise nature of these optional material and levels of incorporation thereof, will depend on the physical form of the skin cleansing composition and the nature of the cleansing operation for which it is to be used. Optional materials can usually be formulated at 6% or less, 5% or less, 4% or less, 3% or less, 2% or less, 1% or less, 0.5% or less, 0.25% or less, 0.1% or less, 0.01% or less, or 0.005% or less by weight of the skin cleansing composition.

To further improve stability under stressful conditions such as high temperature and vibration, the densities of the separate phases can be adjusted such that they can be substantially equal. To achieve this, low density microspheres can be added to one or more phases of the skin cleansing composition. Examples of skin cleansing compositions that comprise low density microspheres are described in a patent application published on May 13, 2004 under U.S. Patent Publication No. 2004/0092415A1 entitled "Striped Liquid Personal Cleansing Compositions Containing A Cleansing Phase and A Separate Phase with Improved Stability," filed on Oct. 31, 2003 by Focht, et al.

The skin cleansing composition can also comprise a benefit component that can be selected from the group consisting of thickening agents; preservatives; antimicrobials; fragrances; chelators (e.g., such as those described in U.S. Pat. No. 5,487,884 issued to Bisset, et al.); sequestrants; vitamins (e.g., Retinol); vitamin derivatives (e.g., tocophenyl actetate, niacinamide, panthenol); sunscreens; desquamation actives (e.g., such as those described in U.S. Pat. No. 5,681,852 and 5,652,228 issued to Bisset); anti-wrinkle/ anti-atrophy actives (e.g., N-acetyl derivatives, thiols, hydroxyl acids, phenol); anti-oxidants (e.g., ascorbic acid derivatives, tocophenol) skin soothing agents/skin healing agents (e.g., panthenoic acid derivatives, aloe vera, allantoin); skin lightening agents (e.g., kojic acid, arbutin, ascorbic acid derivatives) skin tanning agents (e.g., dihydroxyacteone); anti-acne medicaments; essential oils; sensates; pigments; colorants; pearlescent agents; interference pigments (e.g., such as those disclosed in U.S. Pat. No. 6,395,691 issued to Liang Sheng Tsaur, U.S. Pat. No. 6,645,511 issued to Aronson, et al., U.S. Pat. No. 6,759,376 issued to Zhang, et al, U.S. Pat. No. 6,780,826 issued to Zhang, et al.) particles (e.g., talc, kolin, mica, smectite clay, cellulose powder, polysiloxane, silicas, carbonates, titanium dioxide, polyethylene beads) hydrophobically modified non-platelet particles (e.g., hydrophobically modified titanium dioxide and other materials described in a commonly owned, patent application published on Aug. 17, 2006 under Publication No. 2006/0182699A, entitled "Skin cleansing Compositions Containing Hydrophobically Modified Non-platelet particle filed on Feb. 15, 2005 by Taylor, et al.) and mixtures thereof. The skin cleansing compositions can comprise from 0.1% to 4%, by weight of the skin cleansing composition, of hydrophobically modified titanium dioxide. Other such suitable examples of such skin actives are described in U.S. Patent Application Serial No. 13/157,665.

Other optional materials can be those materials approved for use in cosmetics and that are described in the CTFA Cosmetic Ingredient Handbook, Second Edition, The Cosmetic, Toiletries, and Fragrance Association, Inc. 1988, 1992.

### METHODS

### A) DRY SKIN GRADE SCREEN AND APPLICATION OF MATERIALS METHOD

Test subjects are screened for dry skin grade of 2.5-4.0 by trained expert graders following the guidelines below. Prior to the study, subjects participate in a washout period for seven days, in which the subjects only use soap that is provided to them (e.g., soap including shea butter and no beads) and abstain from washing their legs with any other products. Subjects are also instructed to abstain from applying any leave-on products to their legs during the pre-study washout period.

Visual evaluations will be done with the aid of an Illuminated Magnifying Lamp which provides 2.75X magnification and which has a shadow-free circular fluorescent light source (General Electric Cool White, 22 watt 8" Circline). At least 36 subjects are needed to obtain sufficient replicates for each treatment. Table 3 shows a grading scale for dry skin and lists the redness and dryness characteristics associated with each grade.

**Table 3**

| Grade* | Redness | Dryness** |
|---|---|---|
| 0.0 | No redness | Perfect skin |
| 1.0 | Barely detectable redness | Patches of checking and/or slight powderiness, occasional patches of small scales may be seen, distribution generalized |
| 2.0 | Slight redness | Generalized slight powderiness, early cracking, or occasional small lifting scales may be present |
| 3.0 | Moderate redness | Generalized moderate powderiness and/or heavy cracking and lifting scales |
| 4.0 | Heavy or substantial redness | Generalized heavy powderiness and/or heavy cracking and lifting scales |
| 5.0 | Severe redness | Generalized high cracking and lifting scales, eczematous change may be present, but not prominent, may see bleeding cracks |
| 6.0 | Extreme redness | Generalized severe cracking, bleeding cracks and eczematous changes may be present, large scales may be sloughing off |
| *Half-unit grades may be used if necessary | | |
| **"Generalized" refers to situations where more than 50% of an application area is affected | | |

Before initial visual grading, a clinical assistant will mark 2-7 cm (across) x 10 cm (down) treatment sites on an outer portion of the lower legs using a template and a laboratory marking pen (4 comer brackets are sufficient to delineate each area). For assignment of the products, two sites located on the left leg will be numbered L1 and L2, where L1 is the top part of the lower leg nearest the knee, and L2 is the bottom part of the lower leg nearest the ankle. Two sites located on the right leg will be numbered R1 and R2, where R1 is the top part of the lower leg nearest the knee, and R2 is the bottom part of the lower leg nearest the ankle.

To simplify the treatment process, master trays can be prepared for each treatment plan specified in the study randomization. Each master tray can be divided in half, with each half labeled 'left' or 'right' to indicate which leg it corresponds to, then subdivided into sections for the test products in the order of leg application site. One or more make-up trays can also be prepared for use as needed using individual coded containers, or other appropriate product code indicators, that can be re-arranged according to a given treatment plan.

Trained clinical assistants will wash each subject's lower legs in a controlled manner with assigned treatments once daily for 21 consecutive days. Assignment of test treatments to skin sites on the left and right legs will be designated by study randomization. A target dose of body wash for each site is 10 µL/cm². All body wash products will be dispensed at 0.7 mL dosages. All body wash test products will be drawn up into syringes at the 0.7 mL dosage. A one day supply of syringes for all products may be filled the day before or the day of use. Product that has been transferred to another container and the container itself will be used for one day only (i.e., the day the transfer occurred). All syringe filling operations will be appropriately documented (e.g., product code filled, when filled, initials of person responsible for filling).

The treatment area on the top part of the left leg of the subject is wetted for 5 seconds with 95-100°F running tap water. The water flow rate is 1200 mL per minute. For the "No Treatment" site, apply water only. For a treatment site, dispense 0.7 mL of body wash product from the syringe onto the center of the treatment area and place a wet puff over the dispensed product and gently rub the puff back and forth within the treatment site for 10 seconds. Then, allow lather (or water only) to remain on the site for 90 seconds. When residence time for a site has expired, the site is rinsed for 15 seconds under a running tap, taking care not to rinse adjacent sites. After the application area has been rinsed, the area is gently patted dry. Repeat the procedure for the lower part of the left leg, and after completion, use the same procedure for each of the top part of the right leg and the lower part of the right leg.

### B) BIOPHYSICAL MEASUREMENTS AND STRATUM CORNEUM SAMPLING

Measurements of skin hydration can be obtained using a Corneometer CM 825 (Courage + Khazaka Cologne Germany) and TEWL can be measured using a Dermalab^{®} Evaporimeter (Cortex Technologies). Biophysical measurements are made after at least 30 minutes of equilibration in a controlled environment room with temperature (70°F±2) and RH 30-45%. Stratum corneum from the outer aspect of the lower legs is sampled using 10 successive D-Squame^{®} Standard Sampling Discs (D100, CuDerm Corporation, Dallas, TX). Each sampling disc is pressed down onto the site using the D-Squame Pressure Instrument (D500, CuDerm Corporation, Dallas, TX) for 5 seconds, then removed from the skin and placed into 12-well collection plates. The discs can be analyzed for total protein, pyrrolidone carboxylic acid (PCA), interleukin 1α (IL-1α), interleukin 1 receptor agonist (IL-1ra), keratin-1,10,11, and lipids including selected ceramides, selected fatty acids, cholesterol and cholesterol sulfate. Two sites on each leg are sampled and data is averaged at each tape strip for each subject.

When comparing skin in summer versus winter, the same 25 panelists should be remeasured in the summer after going through a 7 day prewash, assessed by visual grading, biophysical measurements, and biomarker analysis done exactly as above. The average outdoor temperature during the winter study was 4.4°C. The average temperature during the summer study was 21.8°C.

### C) D-SQUAME ANALYSIS SCHEME

The protein content of all D-Squame^{®} sampling discs was analyzed nondestructively by measuring the optical absorption with a SquameScan^{®} 850A infrared densitometer (Heiland Electronic, Wetzlar, Germany). The device measures optical absorption at 850 nm which is linearly related to protein content of the D-Squame^{®} sample. Stratum corneum Cytokines (IL-1α and IL-1ra) were measured using tape 2, NMF components were measured using tape 3 and tape 10. Structural proteins (involucrin, keratin 1,10,11) were measured on tape 4 and stratum corneum lipids, like cytokines, were measured using tapes 6 & 7 pooled together for better sensitivity. Measurements for cytokines, NMF and structural proteins were normalized to protein measured by the Pierce^{®} BCA protein assay and lipids were normalized to SquameScan^{™} values.

### D) ANALYSIS OF NATURAL MOISTURIZING FACTORS (NMFs) FROM D-SQUAME^{®} DISCS

NMFs (L-Citrulline, Glycine, L-Omithine, L-Proline, 2-Pyrrolidone-5-carboxylic Acid, L-Serine, trans-Urocanic Acid, and L-Histidine) on D-Squame^{®} discs collected from subjects were prepared for analysis by placing them into 2 mL polypropylene tubes with the glue side facing inwards. A 25 µL aliquot of an internal standard solution (L-Citrulline-D₇; Glycine-D2,¹⁵N; Histidine-D₃; L-Ornithine-D₆; L-Proline-D3: 2-Pyrrolidone-5-carboxylic-D₅ Acid; L-Serine-D3; cis-Urocanic-¹³C₃ Acid) was added to each tube followed by 1.0 mL of water containing 0.1% formic acid and 0.1% heptafluorbutyric acid. The tubes were capped, vortexed for 10 seconds and then placed on a sonicator for 10 min. An aliquot of the extraction solution was removed for analysis by gradient reversed-phase high performance liquid chromatographic (HPLC) analysis on a Waters Atlantis T3 column (2.1 x 50 mm, 3-µm particles). Detection and quantitation was by tandem mass spectrometry (MS/MS, Sciex AB-5000) operating under multiple reaction monitoring (MRM) conditions for each analyte and the corresponding internal standard. Calibration standards (STD) prepared in 1.0 mL of water containing 0.1% formic acid and 0.1% heptafluorbutyric acid were used to generate regression curves for each NMF by plotting the peak area ratio for a given NMF standard (peak area NMF/peak area for internal standard) versus the standard concentration. The concentration of a given NMF in the study samples was determined from its corresponding peak area ratio by interpolation from the regression curve. The nominal range of quantitation is 20 to 20,000 ng/mL (20 to 20,000 ng/tape strip) for each NMF. The concentration of each NMF determined in the acid extract was converted into mass NMF/strip by multiplying by the extraction volume. The found mass of each NMF was then normalized by the protein amount in the acid extract determined by BCA assay (BCA Protein Assay Kit (Pierce Biotechnology/Thermo Scientific, Rockford, IL, USA) using bovine serum albumin as a standard.

### E) ANALYSIS OF INTERLEUKINS IL-1α AND IL-1RA FROM D-SQUAME^{®} DISCS

Human inflammatory cytokines were analyzed to evaluate skin irritation and inflammatory processes. D-Squame^{®} discs collected from subjects were extracted with phosphate-buffered saline (PBS) containing an additional 0.25M NaCl and a commercially available protease inhibitor cocktail containing a mixture of protease inhibitors with broad-spectrum inhibitory specificity (Roche Applied Science, Inc., Indianapolis, IN, USA) for 30 min with sonication on ice. The extracts were then centrifuged for 5 min at 2100 x g to remove skin solids that might interfere in the assay. Aliquots of these extracts were then analyzed for soluble protein using the BCA Protein Assay Kit (Pierce Biotechnology/Thermo Scientific, Rockford, IL, USA) using bovine serum albumin (BSA) as a reference standard. After protein analysis, extracts were supplemented with 2% BSA, transferred into 96-well polypropylene deep-well plates and frozen at -80°C for cytokine analysis. Multiple human cytokines (IL-1α and IL-1ra) were simultaneously quantitated using a Milliplex Human Cytokine Multiplex Immunoassay Kit (Millipore Corp., Billerica, MA, USA).

### F) ANALYSIS OF SKIN PROTEINS FROM D-SQUAME^{®} DISCS

D-Squame^{®} discs were extracted with PBS containing 0.2% sodium dodecyl sulfate (SDS) and 0.5% propylene glycol (PG) for 30 min with sonication on ice. The extracts were then centrifuged for 5 min at 2100 x g to remove skin solids that might interfere in the assay. Subsequently, the extracts of D- Squame^{®} discs were transferred into 96-well polypropylene deep-well plates and frozen at -80⁰C for SkinMAP (multiple analyte profile) and soluble protein analyses. Human skin proteins (Keratin-1, 10; involucrin; human serum albumin (HSA)) were simultaneously quantified using a 3-plex Human Skin Panel Multiplex Immunoassay Kit (Millipore Corp., Billerica, MA, USA). The antibody for human involucrin recognizes non-crosslinked involucrin protein, but may have reactivity with involucrin within the cornified envelope. Soluble protein was measured using BCA Protein Assay Kit (Pierce Biotechnology/Thermo Scientific, Rockford, IL, USA).

### G) ANALYSIS OF SKIN LIPIDS FROM D-SQUAME^{®} DISCS

An array of skin lipids (cholesterol, cholesterol sulfate, selected fatty acids and selected ceramides were determined from extracts of D-Squame^{®} discs sampled from human skin using gradient supercritical fluid chromatography (SFC) with tandem mass spectrometry (MS/MS) with detection in the positive and negative ionization modes depending on the analyte using atmospheric pressure chemical ionization (APCI). The tape strips were first analyzed via a SquameScan^{®} 850A infrared densitometer to determine the amount of removed skin for normalization of the measured lipids. Two tape strips from each subject were transferred to 20 mL glass vials, spiked with an internal standard mixture (D₆-cholesterol, D₇-cholesterol sulfate, D₄₇-tetradecanoic acid, D₃-heptadecanoic acid, D₇-sphinanine and D₃₁-N-palmitoyl-1-D-eryhhro-sphingosine (D₃₁ Ceramide)) and extracted using 3 mL of methanol with sonication at ambient temperature. The vials were centrifuged and the methanol layer removed and placed in separate glass vial. The tape strips were then extracted with 3 mL of hexane with sonication for 15 min at ambient temperature and the hexane layer was isolated. The hexane and methanol layers for each set of tapes were then combined, dried under nitrogen at 50°C and finally reconstituted in chloroform:MeOH (3:1; v/v). Standards (myristic acid, palmitic acid, palmitoleic acid, octadecanoic acid, oleic acid, linoleic acid, docosanoic acid, tetraocosanoic acid, cholesterol, cholesterol sulfate, N(24_0)P(18), N(24_0)DS(18), A(16_0)S(18), A(24_0) P(18), ceramide EOS-C30, S(18)) were prepared in chloroform:MeOH (3:1) over a range of appropriate concentrations. The standards, spiked with internal standard, and the reconstituted samples were analyzed by gradient SFC with MS/MS detection using APCI. The fatty acids were monitored in the negative ion mode while selected ceramides, sphingoid bases, cholesterol and cholesterol sulfate were monitored in the positive ion mode. The peak area ratio (standard peak area/internal standard peak area) for each standard level were used to construct a linear regression curve for each of the standard analytes. For analytes where the standard was available (fatty acids, cholesterol, cholesterol sulfate, sphingoid bases) the actual standard was used, while for the ceramides the surrogate ceramide for the particular class was used. The lipid mass found for each analyte was divided by the Squame Scan values for the corresponding tapes.

### EXAMPLES

The following examples describe and demonstrate examples within the scope of the invention. The examples are given solely for the purpose of illustration and are not to be construed as limitations of the present invention.

| | Inv. Exp. A | Inv. Exp. B |
|---|---|---|
| Surfactant phase | | |
| Water and minors (ex. fragrance) | Q. S. | Q. S. |
| Sodium Trideceth-2 Sulfate | 9.13 | 9.57 |
| Cocoamidopropyl Betaine | 2.73 | 2.86 |
| Sodium Chloride | 4.75 | 4.75 |
| Trideceth-3 | 1.46 | 1.53 |
| Guar hydroxypropyltrimonium chloride (N-Hance CG-17 from Aqualon) | 0.47 | 0.50 |
| Xanthan Gum | 0.32 | 0.34 |
| Sodium Benzoate | 0.30 | 0.30 |
| Methylchloroisothiazolinone / isothiaxolinone (Kathon CG from Rohm & Haas) | 0.037 | 0.037 |
| C10-C30 Alkylacrylates Cross Polymer (Aqupec Ser W-300C from Sumitomo) | 0.03 | 0.03 |
| EDTA | 0.15 | 0.15 |
| Adjust pH to 5.7 with either citric acid or NaOH | | |

| Benefit Phase | | |
|---|---|---|
| Petrolatum | 9.8 | 6.86 |
| Glyceryl monooleate | 0.2 | 0.14 |

Inventive Examples A and B can be prepared through a conventional mixing technique. First, prepare a polymer premix by adding Aqupec SER-300C into Trideceth-3 in a container and separately prepare a citric acid premix in another container (made by adding citric acid power into water at 50:50 w/w ratio). Once the two pre-mixes are completed, add water into the main mixing vessel. Then add sodium chloride, guar hydroxypropyltrimonium chloride, sodium trideceth-2 sulfate, cocamidopropyl betaine, trideceth-3/Aqupec premix (above), xanthan gum, sodium benzoate, and EDTA with continuous mixing. Adjust pH to 5.7 by adding citric acid solution (above) or NaOH solution. Then, add perfume and Kathon. This completes the cleansing phase. Then, add the benefit phase, soybean oil, into the surfactant phase. Keep mixing until homogeneous. This completes the cleansing phase. In a separate lipid container, add petrolatum and heat to 90C, then add glyceryl monooleate into petrolatum container with mixing. Then, add the hot lipid phase into the cleansing phase through controlled static mixing device.
Comparative Product C: Water control (no product treatment)
Comparative Product D: A commercial Dove^{®} Deep Moisture body wash was used as a comparative Product D. The ingredient list is as follows: Water, Cocamidopropyl Betaine, Sodium Hydroxypropyl Starch Phyosphate, Lauric Acid, Sodium Lauroyl Glycinate, Sodium Lauroyl Isethionate, Hydrogenated Soybean Oil, Glycine Soja (Soybean) Oil or Helianthus Annus (Sunflower) Seed Oil, Sodium Chloride, Glycerine, Fragrance and minors.

### Result 1: Total Ceramides

| **Baseline** | Grouping | Total Ceramides (ng/µg protein) |
|---|---|---|
| Inventive Example A | a | 21.03 |
| Inventive Example B | a | 20.18 |
| Comparative Control C: Water Only | a | 21.15 |
| Comparative Control D: Dove Deep Moisture | a | 21.07 |

| At Day 21 | Grouping | Total Ceramides (ng/µg protein) | increase% vs. Control C | increase% vs. Control D |
|---|---|---|---|---|
| Inventive Example A | C | 19.87 | 19.8% | 12.9% |
| Inventive Example B | Bc | 18.53 | 11.7% | 5.4% |
| Comparative Control C: Water Only | A | 16.59 | Control | |
| Comparative Control D: Dove Deep Moisture | Ab | 17.59 | | Control |

### Result 2: Ceramide NP: N28 0 P18

| At Baseline | Grouping | Total Ceramides (ng/µg protein) |
|---|---|---|
| Inventive Example A | a | 4.20 |
| Inventive Example B | a | 4.09 |
| Comparative Control C: Water Only | a | 4.33 |
| Comparative Control D: Dove Deep Moisture | a | 4.20 |

| At Day 21 | Grouping | Ceramide NP (ng/µg protein) | increase% vs. Control C | increase% vs. Control D |
|---|---|---|---|---|
| Inventive Example A | D | 3.85 | 34.3% | 24.0% |
| Inventive Example B | Bed | 3.44 | 19.9% | 10.7% |
| Comparative Control C: Water Only | A | 2.87 | Control C | |
| Comparative Control D: Dove Deep Moisture | Ab | 3.11 | | Control D |

### Result 3: Ceramide NDS: N26_0_DS18

| At Baseline | Grouping | Ceramide NDS (ng/µg protein) |
|---|---|---|
| Inventive Example A | a | 2.30 |
| Inventive Example B | a | 2.23 |
| Comparative Control C: Water Only | a | 2.34 |
| Comparative Control D: Dove Deep Moisture | a | 2.36 |

| At Day 21 | Grouping | Ceramide NDS (ng/µg protein) | increase% vs. Control C | increase% vs. Control D |
|---|---|---|---|---|
| Inventive Example A | C | 2.41 | 16.9% | 14.8% |
| Inventive Example B | Bc | 2.30 | 11.6% | 9.5% |
| Comparative Control C: Water Only | A | 2.06 | Control C | |
| Comparative Control D: Dove Deep Moisture | Ab | 2.10 | | Control D |

### Result 4: Ceramide NP: N24 0 P18

| At Baseline | Grouping | Ceramide NP (ng/µg protein) |
|---|---|---|
| Inventive Example A | ab | 6.02 |
| Inventive Example B | a | 5.69 |
| Comparative Control C: Water Only | ab | 6.07 |
| Comparative Control D: Dove Deep Moisture | ab | 6.13 |

| At Day 21 | Grouping | Ceramide NP (ng/µg protein) | increase% vs. Control C | increase% vs. Control D |
|---|---|---|---|---|
| Inventive Example A | D | 5.00 | 38.4% | 29.7% |
| Inventive Example B | Bc | 4.38 | 21.2% | 13.6% |
| Comparative Control C: Water Only | A | 3.61 | Control C | |
| Comparative Control D: Dove Deep Moisture | Ab | 3.85 | | Control D |

## Claims

1. A method of screening cleansers for mildness, wherein mildness results in enhancing selective ceramides in the stratum corneum for enhanced skin barrier function and hydration comprising:
a) measuring the level of one or more ceramides on an area of skin prior to application of a cleanser, wherein the ceramide comprises N₂₄P₁₈, N₂₆DS₁₈, or N₂₈P₁₈;;
b) applying the cleanser to the area of skin for 21 days, wherein 0.07mL or more of the cleanser is applied during each application, wherein the cleanser comprises sodium trideceth-2 sulfate, cocamidopropyl betaine, guar hydroxypropyltrimonium chloride, a C10-C30 acrylate crosspolymer, and a benefit agent;
c) measuring the level of one or more ceramides after the product application of 21 days on the area of skin, according to the Analysis of Skin Lipids from D-Squame Discs as disclosed herein;
wherein the cleanser is mild if the level of the one or more ceramides is at least 10%vs. the no treatment control.

2. The method of claim 1, wherein the cleanser is applied in accordance with the Dry Skin Grade Screen and Application of Materials Method as disclosed herein.

## Patentansprüche

1. Verfahren zum Screenen von Reinigern auf Milde, wobei Milde zum Verbessern selektiver Ceramide in dem Stratum Corneum für eine verbesserte Hautbarrierefunktion und Hydratation führt, umfassend:
a) Messen des Gehalts eines oder mehrerer Ceramide auf einem Hautbereich vor einer Anwendung eines Reinigers, wobei das Ceramid N₂₄P₁₈, N₂₆DS₁₈ oder N₂₈P₁₈ umfasst;
b) Anwenden des Reinigers auf den Hautbereich für 21 Tage, wobei 0,07 mL oder mehr des Reinigers bei jeder Anwendung angewendet werden, wobei der Reiniger Natriumtrideceth-2-sulfat, Cocamidopropylbetain, Guargummihydroxypropyltrimoniumchlorid, ein C10-C30-Acrylatckreuzpolymer und einen Wirkstoff umfasst;
c) Messen des Gehalts eines oder mehrerer Ceramide nach der Produktanwendung von 21 Tagen auf dem Hautbereich, nach der Analyse von Hautlipiden aus D-Squame-Plättchen, wie hierin offenbart;
wobei der Reiniger mild ist, falls der Gehalt des einen oder der mehreren Ceramide wenigstens 10 % gegenüber der Kontrolle ohne Behandlung beträgt.

2. Verfahren nach Anspruch 1, wobei der Reiniger gemäß dem Trockene-Haut-Grad-Screening-und-Materialanwendungsverfahren, wie hierin offenbart, angewendet wird.

## Revendications

1. Procédé de dépistage de nettoyants pour la douceur, dans lequel la douceur entraîne un renforcement des céramides sélectifs dans la stratum corneum pour améliorer la fonction de barrière cutanée et l'hydratation, comprenant :
a) la mesure du niveau d'un ou plusieurs céramides sur une zone de peau avant l'application d'un nettoyant, dans lequel le céramide comprend N₂₄P₁₈, N₂₆DS₁₈, ou N₂₈P₁₈ ;
b) l'application du nettoyant sur la zone de peau pendant 21 jours, dans lequel 0,07 mL ou plus du nettoyant est appliqué lors de chaque application, dans lequel le nettoyant comprend du tridéceth-2 sulfate sodique, de la cocamidopropylbétaïne, de la chlorure d'hydroxypropyltrimonium de guar, un polymère croisé de acrylate en C10 à C30, et un agent bénéfique ;
c) la mesure du niveau d'un ou plusieurs céramides après l'application du produit de 21 jours sur la zone de la peau, selon l'analyse des lipides cutanés des disques D-Squame telle que décrite ici ;
dans lequel le nettoyant est doux si le taux du ou des céramides est d'au moins 10 % par rapport au témoin sans traitement.

2. Procédé selon la revendication 1, dans lequel le nettoyant est appliqué conformément au procédé de criblage de qualité peau sèche et au procédé d'application de matériaux tel que décrit ici.
